# EUROPEAN PATENT SPECIFICATION

(11) **EP 3 239 712 B1**
(45) Date of publication and mention of the grant of the patent: **10.03.2021**
(21) Application number: 16167699.4
(22) Date of filing: 29.04.2016
(51) Int. Cl.: G01N 33/68

(54) **METHOD FOR DETERMINING THE ORIGIN OF AN INFECTION**
VERFAHREN ZUR ERMITTLUNG DES URSPRUNGS EINER INFEKTION
PROCÉDÉ DE DÉTERMINATION DE L'ORIGINE D'UNE INFECTION

(43) Date of publication of application: 01.11.2017
(73) Proprietor: Universitätsklinikum Jena, 07747 Jena (DE)
(72) Inventor: Kiehntopf, Michael, 07743 Jena (DE); Schmerler, Diana, 07743 Jena (DE)
(74) Representative: CH Kilger Anwaltspartnerschaft mbB

(56) References cited:
- WO-A1-2013/072381
- AILKO W. J. BOSSINK ET AL: "Prediction of Microbial Infection and Mortality in Medical Patients with Fever: Plasma Procalcitonin, Neutrophilic Elastase-[alpha]-Antitrypsin, and Lactoferrin Compared with Clinical Variables", CLINICAL INFECTIOUS DISEASES, vol. 29, no. 2, 1 August 1999 (1999-08-01) , pages 398-407, XP055282050, US ISSN: 1058-4838, DOI: 10.1086/520222
- NANCY BLAUROCK ET AL: "C-Terminal Alpha-1 Antitrypsin Peptide: A New Sepsis Biomarker with Immunomodulatory Function", MEDIATORS OF INFLAMMATION., vol. 90, no. 9, 1 January 2016 (2016-01-01), pages 3356-13, XP055282177, GB ISSN: 0962-9351, DOI: 10.1097/01.ccm.0000130997.85379.0f

## Description

### Field of Invention

The present invention is in the field of medicine. It relates to a method and kit for determining whether an infection is of bacterial or non-bacterial origin.

### Background

Antibiotic resistance due to the inappropriate use of antimicrobials is one of the most critical public health problems worldwide. A major factor underlying the unnecessary use of antibiotics is the lack of rapid and accurate diagnostic tests. The early identification of bacterial infections and its differentiation from other types of infection is still a challenge for clinicians.

This is mainly due to the fact that the clinical presentation of infections from different causative agents can be very similar. For example, in certain instances it may be difficult to differentiate bacterial from viral or fungal infections. Although untreated bacterial infections may cause serious complications, treating viral illnesses or non-infective causes with antibiotics is not only ineffective, but also contributes to the development of resistance, increases costs, and adds the risks of toxicity and allergic reactions. Studies undertaken by the World Health Organization indicate that, for every 100 respiratory infections, only 20 require antibiotic treatment. It is estimated that physicians in Canada and the United States overprescribe antibiotics by 50%.

The most precise way to diagnose bacterial infections is by culture. Tests to confirm viral infections include determination of acute- and convalescent-phase antibody titers and tests for viral antigens. However, there is often a delay until results are known, and rapid immunological or genomic tests require prior knowledge of the infectious agent. Theoretically, biomarkers would be the ideal choice for the early recognition of bacterial infections, for the guidance of treatments, reduction of misuse of antibiotics, and possibly improvement of long-term outcomes. However, the markers currently available still have various drawbacks.

Among others, procalcitonin (PCT) and C-reactive protein (CRP) markers are commonly used for diagnosing a bacterial infection and/or differentiating between different types of infection or systemic inflammatory reactions. PCT is the pre-hormone of calcitonin, which is normally secreted by the C cells of the thyroid in response to hypercalcemia. Under these normal conditions, negligible serum PCT concentrations are detected. The mechanism proposed for PCT production after inflammation and its role are still not completely known but it is believed that PCT is produced by the liver and peripheral blood mononuclear cells, modulated by lipopolysaccharides and sepsis-related cytokines. CRP is an acute-phase reactant, and CRP level measurements are frequently used to aid in the diagnosis of bacterial infections. CRP is synthesized by the liver, mainly in response to IL-6, which is produced not only during infection but also in many types of inflammation. It binds to polysaccharides in pathogens, activating the classical complement pathway.

Although PCT and CRP-Tests are prevalent in clinical settings, their accuracy in diagnosing a bacterial infection is heavily questioned and varies between different studies. Also, the clinical benefit for diagnostics and monitoring of therapy is debated as costs for tests are very high.

Therefore, there is a clear need for new markers and methods with improved diagnostic characteristics. The object of the present invention is to provide new suitable markers, methods and means for differentiating a bacterial from a non-bacterial infection with high sensitivity and specificity.

### Summary of the invention

The inventors surprisingly found that specific concentrations of antitrypsin (ATT) and/or fragments thereof in samples of patients suffering from an infection, are indicative of the origin of the infection.

One aspect of the present invention therefore relates to a method for determining in a patient whether an infection is of bacterial or non-bacterial origin, the method comprising the steps of (a) determining the concentration of at least one antitrypsin (ATT) fragment comprising CAAP48 and/or CAAP47, in a sample that has been obtained from said patient, and (b) determining whether said patient suffers from an infection of bacterial or non-bacterial origin based on the concentration of at least one ATT fragment comprising CAAP48 and/or CAAP47 and designating the infection as of bacterial origin or non-bacterial origin, wherein said non-bacterial infection is selected from the group comprising viral and fungal infections.

The invention further relates to a kit comprising the means for determining whether an infection is of bacterial or non-bacterial origin, comprising (a) the means for determining the concentration of at least one ATT fragment comprising CAAP48 and CAAP47 in a sample, and (b) the means for comparing the determined concentration of said at least one ATT fragment comprising CAAP48 and CAAP47 with reference concentrations, as well as (c) instructions for carrying out the method and correlating the determined concentrations with the type of infection.

### Definitions

In the context of the present application, the term "fragment" refers to a proteolytic cleavage product of a respective full-length protein.

The term "patient" as used herein refers to a living human or non-human organism that is receiving medical care or that should receive medical care due to a disease, or is suspected of having a disease. This includes persons with no defined illness who are being investigated for signs of pathology. Thus the methods and assays described herein are applicable to both, human and veterinary disease.

Herein, **"alpha-1-antitrypsin"** is also referred to as antitrypsin and "ATT". Alpha 1 Antitrypsin or α-1-antitrypsin (A1AT) is a protease inhibitor belonging to the serpin superfamily. It is generally known as serum trypsin inhibitor. Alpha-1-antitrypsin is also referred to as alpha-1-proteinase inhibitor (A1PI) because it inhibits a wide variety of proteases. It protects tissues from enzymes of inflammatory cells, especially neutrophil elastase, and has a reference range in blood of 1.5 - 3.5 g/l, but the concentration can rise manifold upon acute inflammation.

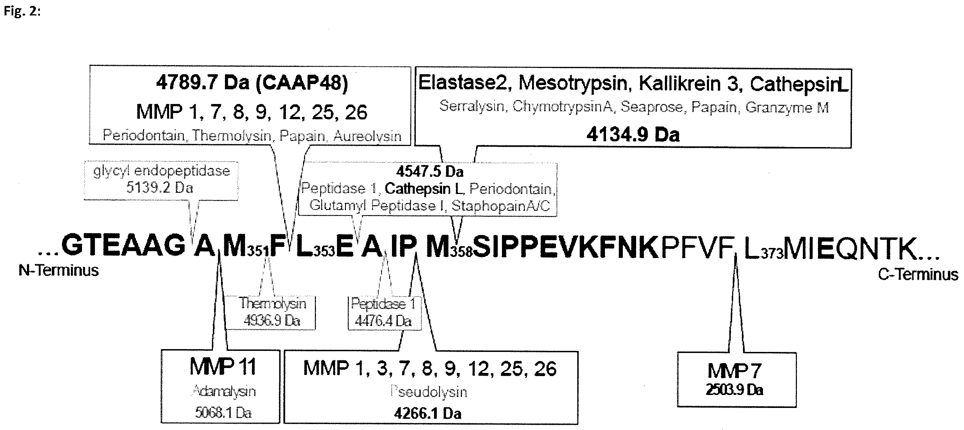

ATT is cleaved at the reactive center loop (RCL) by target proteases (e.g. neutrophil elastase), as well as non-target proteases (e.g. matrix metalloproteases) (Fig.2). Both reactions lead to a conformational change and a loss of inhibitory activity.

**C-terminal alpha-1 antitrypsin peptide** is herein referred to as **CAAP48.** CAAP48 is generated through the cleavage of AAT between Phe352-Leu353 and results in a carboxyl-terminal 42-residue peptide with a mass of 4789 Da, which is bound to the cleaved complex in a non-covalent manner (Fig. 2). Variants of this fragment such as VIRIP or C-36 peptide have been identified in several human tissues and body fluids. For these C-terminal fragments physiological functions, such as NK-cell suppression and serine protease protection (CRISPP peptide), extracellular matrix protection by reversible serine protease inhibition (SPAAT), pro- or anti-inflammatory immune modulating functions (C-36 peptide) as well as inhibition of HIV entry (VIRIP) have been reported.

**CAAP47** is a SNP variant of CAAP48, resulting from a E > D substitution. In the AAT gene over 75 polymorphisms have been described and one frequent SNP (rs1303, MAF = 0.28) occurs in the sequence of CAAP48. Cleavage of AAT between Phe352-Leu353 in the presence of SNP rs1303 leads to the generation of a cleavage product with a lower molecular mass of 4775 Da, designated CAAP47 (Fig.2).

In the context of the present invention, the terms "threshold", "threshold value", "cut-off" and "cut-off value" are used synonymously. The optimal cut-off value is defined to be the concentration at which patients are classified into the bacterial or non-bacterial group with the highest sensitivity and specificity.

As used herein, a "kit" is a packaged combination optionally including instructions for use of the combination and/or other reactions and components for such use.

### Detailed description of the invention

The timely discrimination of a bacterial from a non-bacterial infection is essential for the initiation of an early goal directed therapy. To date there is no reliable biomarker available which facilitates this diagnosis and allows for accurate discrimination between bacterial and non-bacterial infections.

Concentration measurements of AAT and its fragments, in combination with the mindful analysis carried out by the inventors, surprisingly revealed an increased proteolytic activity in patients with bacterial infection resulting in high peak intensities of AAT fragments compared to infections of non-bacterial origin.

The present invention therefore relates to a method for determining in a patient whether an infection is of bacterial or non-bacterial origin, the method comprising determining the concentration of antitrypsin (ATT) and/or of at least one fragment thereof, in a sample that has been taken from said patient, and determining whether said patient suffers from an infection of bacterial or non-bacterial origin based on the concentration of ATT and/or of at least one fragment thereof and designating the infection as of bacterial or non-bacterial origin.

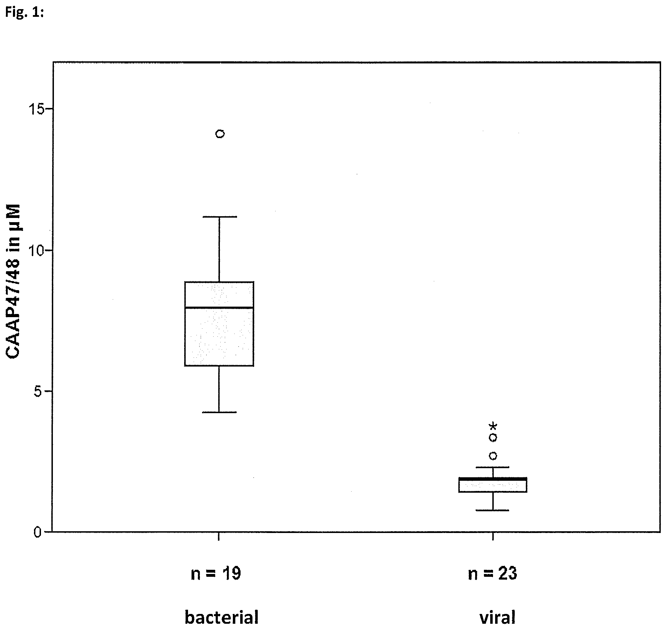

Surprisingly, the inventors found that ATT fragments are substantially elevated in patients suffering from a bacterial infection, compared to patients with a non-bacterial infection (Fig. 1).

Thus, in the method according to the invention, an infection is designated as of bacterial origin if the concentration of at least one ATT fragment is above a certain cut-off value. The optimal cut-off value is defined to be the concentration at which patients are classified into the bacterial or non-bacterial group with the highest sensitivity and specificity.

In the method according to the invention, the infection is designated as of bacterial origin, if the concentration of said at least one ATT fragment is at least 2.5 -fold higher in the patient than a reference concentration. Preferably, the concentration is at least 3 -fold higher, more preferred, at least 4 -fold higher and most preferred at least 5 -fold higher than the reference concentration of said at least one ATT fragment thereof.

A reference concentration in the meaning of the present invention is the median concentration of respective ATT and/or fragments thereof in healthy subjects.

In the method according to the invention, an infection is designated to be of bacterial origin if the concentration of said at least one ATT fragment is between 2.5 to 7 -fold higher than the reference concentration, more preferably between 3 to 6.5 -fold higher, more preferably between 3.5 to 6 -fold higher, preferably between 4 to 5.5 -fold higher, and most preferably between 4.5 to 5 -fold higher than the reference concentration.

In one embodiment of the method according to the invention, the protein and/or fragment concentration is measured via mass spectrometry. Preferably, the concentrations of said ATT and/or fragments thereof are determined via LC-MS/MS. One embodiment of the invention used to quantify said ATT and/or fragments thereof is detailed in the examples.

The surprising difference in concentrations between patients suffering from bacterial and non-bacterial infection could not have been expected beforehand and offers a tremendous diagnostic potential. It can lead to a significantly higher sensitivity and specificity for the diagnosis of bacterial infections than conventional markers commonly used in clinical settings.

Therefore, the method of the invention can also be used to differentiate between several types of infection and determine their origin. In the method according to the invention, said non-bacterial infection is selected from the group comprising viral and fungal infections.

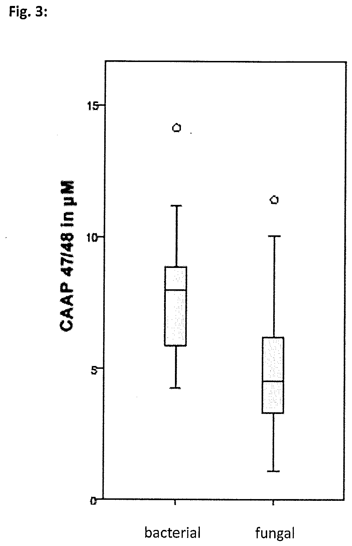

The inventors surprisingly found that the concentration of said at least one ATT fragment, is clearly lower in patients suffering from a viral infection than in patients suffering from a bacterial infection (Fig. 1). The inventors also found that the concentration of said at least one ATT fragment, is also substantially lower in patients suffering from a fungal infection than in patients suffering from a bacterial infection (Fig. 3).

Consequently, the present invention relates to a method wherein, if the concentration of said at least one ATT fragment is below a certain cut-off value, the infection is designated as of non-bacterial origin. Said non-bacterial origin can be, but is not limited to viral origin or fungal origin.

In the method according to the invention, the infection is designated as of non-bacterial origin if the concentration of said at least one ATT fragment is not more than 2.5. -fold higher than a reference concentration. Preferably, said concentration of the at least one ATT fragment is not more than 2 -fold higher than a reference concentration, most preferably not more than 1.5 -fold higher than a reference concentration.

In the method according to the invention, an infection is also designated to be of non-bacterial origin if the concentration of said at least one ATT fragment is between 1.2 to 2.4 -fold higher than the reference concentration, preferably between 1.5 to 2.3-fold higher, most preferably between 1.75 to 2 -fold higher than the reference concentration.

Moreover, in the method according to the invention, said sample taken from a patient is selected from a group comprising a plasma sample, a serum sample, a whole blood sample, a blood sample or fractions thereof, a lymphatic fluid sample, a urine sample and an extract of any of the aforementioned samples.

From the ATT fragment analyzed, the inventors surprisingly identified by using mass-spectrometry **CAAP48/47** (Fig. 2) as potential discriminatory biomarker.

The inventors developed a LC-MS/MS method for quantification of CAAP48 and the SNP-variant CAAP47. Therefore, the method according to the invention also relates to a method in which the concentration of CAAP48 and the SNP-variant CAAP47 is quantified via LC-MS/MS as detailed in the examples. However, the method of quantification of said ATT and/or fragments thereof, such as CAAP48 and/or CAAP47, is not limited to mass spectrometry.

Figure 1 and 3 show the unexpected results obtained by the inventors, that the CAAP47/48 concentrations are significantly elevated in the blood of patients suffering from a bacterial infection compared to patients suffering from a non-bacterial infection.

Therefore, in a method according to the invention for determining in a patient whether an infection is of bacterial or non-bacterial origin, said ATT and/or at least one fragment thereof comprises CAAP48 and/or CAAP47.

Preferably, said ATT and/or at least one fragment thereof is CAAP48 and/or CAAP47.

The present invention therefore relates to a method for determining in a patient whether an infection is of bacterial or non-bacterial origin, the method comprising determining the concentration of CAAP48 and/or CAAP47, in a sample that has been taken from said patient, and determining whether said patient suffers from an infection of bacterial or non-bacterial origin based on the concentration of CAAP48 and/or CAAP47 and designating the infection as of bacterial or non-bacterial origin.

Thus, in the method according to the invention, an infection is designated as of bacterial origin if the concentration of CAAP48 and/or CAAP47 is above a certain cut-off value. The optimal cut-off value is defined to be the concentration at which patients are classified into the bacterial or non-bacterial group with the highest sensitivity and specificity.

In the method according to the invention, the cut-off value for determining that an infection is of bacterial origin is a concentration of CAAP48 and/or CAAP47 of at least 5 µM in the blood of said patient and may deviate depending on the patient analyzed by about 20 %.

Preferably, the cut-off value is at least 5 µM, but it can also be at least 6 µM, at least 7 µM, or at least 8 µM.

Also preferred in a method according to the invention is that the cut-off value for determining that an infection is of bacterial origin is between 4.5 and 8 µM, preferably between 5 and 7.5 µM, more preferably between 5 and 7 µM, also preferably between 5.5 and 6.5 µM between 5.5 and 6 µM, and most preferably between 5 and 5.5 µM.

Moreover, the invention also relates to a method in which, if the concentration of said CAAP48 and/or CAAP47 is at least 2.5 -fold higher than a reference concentration, the infection is designated of bacterial origin. Preferably, the concentration is at least 3 -fold higher, even more preferred, at least 4 -fold higher, and most preferably at least 5 -fold higher than the reference concentration of said CAAP48 and/or CAAP47.

Furthermore, in the method according to the invention, an infection is designated to be of bacterial origin if the concentration of said CAAP48 and/or CAAP47 is between 2.5 to 7 -fold higher than the reference concentration, more preferably between 3 to 6.5 -fold higher, more preferably between 3.5 to 6 -fold higher, more preferably between 4 to 5.5 -fold higher most preferred between 4.5 to 5 -fold higher than the reference concentration.

The CAAP47/48 concentrations are significantly higher in patients suffering from a bacterial infection compared patients suffering from a viral infection. The median concentration of CAAP47/48 in the blood of patients with a bacterial infection is much higher compared to patients with a viral infection (Fig. 1). For example, the inventors found that the median concentration of CAAP47/48 in the blood of patients with a bacterial infection is 7.97 µM, which is a 4-fold increase compared to patients with a viral infection (Fig. 1).

Moreover, the inventors found that the concentration of CAAP47/48 in the blood of patients suffering from a bacterial infection is significantly higher compared patients suffering from a fungal infection. The inventors found that the median concentration of CAAP47/48 in patients suffering from a fungal infection is 4.5 µM, compared to 7.97 µM in patients suffering from a bacterial infection (Fig. 3).

Therefore, in one embodiment of the method according to the invention, the cut-off value for determining that an infection is of non-bacterial origin is a concentration of CAAP48 and/or CAAP47 of less than 5 µM in the blood of said patient and may deviate depending on the patient analyzed by about 20 %.

Preferably, said cut-off value for determining that an infection is of non-bacterial origin is less than 5 µM, preferably less than 4.5 µM, more preferably less than 4 µM.

Also preferred in a method according to the invention, the cut-off value for determining that an infection is of non-bacterial origin is between 3.5 and 5 µM, between 3 and 4.5 µM, or between 3.5 and 4 µM.

As mentioned before, the inventors surprisingly found that the concentration of at least one ATT fragment, is clearly lower in patients suffering from a viral or a fungal infection than in patients suffering from a bacterial infection (Fig. 1 and 3). In addition, and very surprisingly, the inventors found that the concentration of at least one ATT fragment in patients suffering from a fungal infection is in between the concentration of the at least one ATT fragment in patients suffering from a bacterial and the concentration in patients suffering from a viral infection. Therefore, in one embodiment, the method according to the invention allows not only for the discrimination between bacterial and non-bacterial infections, but also whether a non-bacterial infection is of viral and fungal origin.

In one embodiment of the invention, an infection is designated as of fungal origin if the concentration of said at least one ATT fragment is above the cut-off value for designating an infection a viral infection and below the cut-off value for designating an infection as of bacterial origin.

The disclosure herein also relates to a method wherein the infection is designated as to be of fungal origin if the concentration of said at least one ATT fragment is at least 1.5 -fold higher but not more than 2.5. -fold higher than a reference concentration. In another embodiment according to the invention, an infection is designated as to be of viral origin if the concentration of said at least one ATT fragment is not more than 1.5 -fold higher than a reference concentration.

As Figure 1 shows, the CAAP47/48 concentrations are significantly higher in patients suffering from a bacterial infection compared patients suffering from a viral infection.

Therefore, the disclosure herein also relates to a method wherein the cut-off value for determining that an infection is of viral origin is a concentration of CAAP48 and/or CAAP47 of less than 3 µM in the blood of said patient and may deviate depending on the patient analyzed by about 20 %.

Preferably, the cut-off value for determining that an infection is of viral origin is less than 3 µM, more preferably less than 2 µM and most less than 1.5 µM.

Also preferred in a method according to the invention is that the cut-off value for determining that an infection is of viral origin is between 1 and 3 µM, more preferably between 1.5 and 2.5 µM and most preferably 2 µM.

The disclosure herein also relates to a method wherein the infection is designated as of viral origin, if the concentration of said CAAP48 and/or CAAP47 is less than 1.5 fold higher than a reference concentration. Preferably, said concentration to designate an infection as of viral origin is about the same value as the reference concentration of said CAAP48 and/or CAAP47.

As Figure 1 and 3 show, the CAAP47/48 concentrations are significantly higher in patients suffering from a bacterial infection compared patients suffering from a fungal infection and even higher than in patients suffering from a viral infection. The inventors found that the median concentration of CAAP47/48 in the blood of patients with a fungal infection is 4.5 µM.

The disclosure herein also relates to a method wherein an infection is designated as of fungal origin if the concentration of CAAP47/48 is below the cut-off value of designating an infection as to be of bacterial origin and above the cut-off value of designating an infection as to be of viral origin.

Therefore, the disclosure herein also relates to a method wherein an infection is designated as to be of fungal origin if the concentration of CAAP48 and/or CAAP47 in the blood of said patient is between 1.5 µM and 5 µM and may deviate depending on the patient analyzed by about 20 %. Preferably, the concentration of CAAP48 and/or CAAP47 for designating an infection as to be of fungal origin is between 5 µM and 1.5 µM, preferably between 2 µM and 4.5 µM, more preferably between 2.5 µM and 4 µM and most preferably between 3 µM and 3.5 µM.

The disclosure herein further relates to a method wherein the infection is designated as of fungal origin, if the concentration of said CAAP48 and/or CAAP47 is less than 2.5 -fold higher than a reference concentration but more than 1.5 -fold higher than a reference concentration. Preferably, the infection is designated as of fungal origin if the concentration of CAAP47/48 is 2 -fold higher than a reference concentration.

In a further embodiment of the invention, a sample is taken at one or more of the following time points: when the subject is first admitted to a medical institution or in the ambulance, when the subject is in the emergency room, when the subject is in the intensive care unit, before treatment, after initiation of treatment, 24 hours after initiation of treatment, 48 hours after initiation of treatment and/or 72 hours after initiation of treatment.

Therefore, the method according to the invention can also be used to monitor the success of treatment of a patient suffering from an infection. In particular, it can be used to monitor the success of antimicrobial treatment, e.g. with antibiotics.

The disclosure herein also relates to a method for monitoring the success of treating a patient suffering from an infection with a certain drug, comprising the steps of determining the concentration of antitrypsin (ATT) and/or of at least one fragment thereof, in a sample that has been taken from said patient, and associating the levels of ATT and/or fragments thereof with a positive or a negative response to said certain drug.

Said positive response is given if the level of ATT increases during drug treatment and/or if the level of ATT fragments decreases during drug treatment. In one embodiment, said drug can be an antimicrobial drug, e.g. an antibiotic. Said ATT fragments may comprise CAAP47/48.

The disclosure herein also relates to a method for monitoring the success of treating a patient that suffering from an infection with a drug, comprising the steps of:
- taking at least two samples from said patient at various time points selected from the group of,
   i. before initiation of therapy and/or
   ii. after initiation of therapy, and/or
   iii. at one or more further time point
- determining the concentration of ATT and/or fragments thereof in said sample taken from said patient,
- associating the levels of ATT and/or fragments thereof in said samples taken from said patient with a positive or a negative response to said certain drug.

Said positive response is given if the level of ATT increases during drug treatment and/or if the level of ATT fragments decreases during drug treatment. In one embodiment, said drug can be an antimicrobial drug, e.g. an antibiotic. Said ATT fragments may comprise CAAP47/48.

Furthermore, the method according to the invention can be used to determine the appropriate treatment and/or therapy. For example, it can be used to determine the right medication before and during treatment. Therefore, the method according to the invention may prevent harmful side-effects, toxicity and allergic reactions due to inappropriate drug treatment and/or therapy. Moreover, serious complications can be prevented in patients and the healing process can be accelerated. This may also significantly reduce the costs of therapy. In case of treating a patient suffering from an infection, the inappropriate prescription of antibiotics may be prevented, thus preventing the spread of antibiotic resistance.

Thus, the disclosure herein relates to a method of medical decision making for individual patient therapy in a subject suffering from an infection, comprising the steps of, determining the concentration of antitrypsin (ATT) and/or of at least one fragment thereof, in a sample that has been taken from said patient, and associating the levels of ATT and/or fragments thereof with a certain choice of therapy.

In particular, the method according to the invention can be used in medical decision making for individual patient therapy in a subject suffering from an infection. As example, it can be used to determine whether a patient should be treated with antibiotics.

The disclosure herein also relates to a method of medical decision making for individual patient therapy in a subject related to the severity of the disease.

The disclosure herein relates to a method of medical decision making for individual patient therapy related to the severity of the disease by monitoring therapy response in a subject to a certain drug. Such drug can be an antimicrobial-drug, e.g. antibiotics.

Therefore, the disclosure herein also relates to a method of medical decision making for individual patient therapy in a subject suffering from an infection, comprising the steps of,
- taking at least one sample from said subject at various possible time points selected from the group of,
   i. before initiation of therapy and/or
   ii. after initiation of therapy, and/or
   iii. at one or more further time point
- determining the concentration of ATT and/or fragments thereof in said sample taken from said subject,
- associating the levels of ATT and/or fragments thereof in said samples taken from said subject with a positive or a negative response to said therapy.

Said positive response to therapy is given if the level of ATT increases during drug treatment and/or if the level of ATT fragments decreases during drug treatment. In one embodiment of said method of medical decision making for individual patient therapy in a subject suffering from an infection, said therapy may comprise the treatment with antimicrobial drugs, which can be antibiotics. Said ATT fragments may comprise CAAP47/48.

Furthermore, the present invention also relates to a kit for determining whether an infection is of bacterial or non-bacterial origin, comprising means for determining the concentration of ATT and/or of at least one fragment thereof in a sample, means for comparing the determined concentration of said ATT and/or of at least one fragment thereof with reference concentrations, and instructions for carrying out the method and relating the determined concentrations with the type of infection.
In a preferred embodiment, the means for determining the concentration of said ATT and/or of at least one fragment thereof, is an antibody specific for ATT and/or for at least one fragment thereof.

Further preferred, said fragment is CAAP48 and/or CAAP47 and said antibody is specific for CAAP48 and/or CAAP47.

In another embodiment, the means for comparing the determined concentrations comprise standard data showing the correlation between the concentration of said ATT and/or at least one fragment thereof contained in samples and the origin of infection.

The present invention also relates to a kit as described above, wherein said infection of non-bacterial origin is an infection selected from the group comprising viral and fungal infections.

The present invention also relates to a kit according to the invention, wherein said sample is selected from a group comprising a plasma sample, a serum sample, a whole blood sample, a blood sample or fractions thereof, a lymphatic fluid sample, a urine sample and an extract of any of the aforementioned samples.

Due to the easy and low-costs, the method and kit according to the invention can be used not only for determining if an infection is of bacterial or non-bacterial origin, but also for the monitoring of treatment of a patient. Such treatment may comprise the treatment of an infection such as a bacterial, viral or fungal infection. The monitoring of treatment can relate to the monitoring of treatment of a patient with an antimicrobial drug, such as antibiotics.

The disclosure herein also relates to a kit for diagnosis of a bacterial infection and/or predicting the outcome of treatment with a certain drug and/or the monitoring of treatment of a patient suffering from an infection comprising, an antibody, an aptamer, or another target specific molecule specific for ATT and/or fragments thereof, standard data showing the correlation between the amounts of ATT and/or fragments thereof contained in samples and prognosis, and a manual.

Also the disclosure herein relates to an LC-MS/MS kit for diagnosing a bacterial infection and/or predicting the outcome of treatment in a patient with a certain drug and/or the monitoring of treatment of a patient suffering from an infection, wherein the concentrations of ATT and/or fragments thereof are re guaranteed by LC-MS/MS. These concentrations are compared to standard data sets.

### Examples

The following examples are used in conjunction with the figures and tables to illustrate the invention.

### Example 1: Patient groups and characteristics

Four different patient groups were enrolled in the study (non-bacterial and non-viral infections (n = 36), severe sepsis (n = 19), Candida infected patients (n=18) and HIV infected patients (n = 23)).

Nine, six and eight HIV patients were enrolled with virus titers of <100, 100-10000 and > 10000 cop/ml, respectively. Healthy volunteers were included for AAT genotype and phenotype correlation and for the cell experiments. Patients less than 18 years old, pregnant or lacking informed consent were excluded. The study and all protocols were approved by the local ethics committee.

### Example 2: Peptides

Peptides were synthesized by solid phase peptide synthesis and purified by HPLC with greater 95% purity. In short, peptides were synthesized automatically by a standard Fmoc [N-(9-fluorenyl)methoxycarbonyl] protocol by manual SPPS or on an EPS221 peptide synthesizer (Intavis Bioanalytical Instruments AG, Cologne, Germany). In general, coupling reactions were performed using Fmoc-amino acids (5 equiv) activated with HBTU (5 equiv) in the presence of N-methylmorpholine/DMF (1:1) for 5-15 min (double couplings). Fmoc removal was effected by treating the resin twice with piperidine in DMF (20 %). All CAAP48 an immunomodulatory sepsis biomarker deprotection and coupling steps were followed by intensive washings with DMF and dichlorometane (CH2CI2), alternately. Peptide cleavage and deprotection was accomplished with reagent K (trifluoroacetic acid (TFA)/water/phenol/thioanisole/ethanedithiol 82.5:5:5:5:2.5) for 4 h at room temperature. The crude peptides were precipitated with diethyl ether, centrifuged, and washed several times with diethyl ether. The yields of the crude peptides varied between 60 - 70 %. The crude peptides were purified by semipreparative reversed-phase HPLC with a Shimadzu LC-8A system equipped with a C18 column (Knauer Eurospher 100, Berlin, Germany). The gradient elution system was 0.1 % TFA in water (eluent A) and 0.1 % TFA in acetonitrile/water (9:1, eluent B). The peptides were eluted with a gradient of 0 - 50 % eluent B in 120 min and a flow rate of 10 ml/min. The peaks were detected at 220nm. Collected fractions were combined, freeze-dried, and stored at -20°C. Purities of the peptides were confirmed by analytical reversed-phase HPLC with a Shimadzu LC-10AT chromatograph (Duisburg, Germany) equipped with a Vydac 218TP54 column (C18, 5 µm particle size, 300 Å pore size, 4.6x25 mm). The peptides were analyzed by gradient elution at a flow rate of 1 ml/min, where eluent A was 0.1 % TFA in water, and eluent B was 0.1 % TFA in acetonitrile; detection was at 220nm.

### Example 3: Peptide quantification

We developed a LC-MS/MS method for quantification of CAAP48 and the SNP-variant CAAP47 and determined its concentration in the critically ill. Because of divergent characteristics of synthetic and endogenous CAAP48, two plasma samples with defined CAAP48 concentrations were used as standards for quantification by two-point calibration. This LC-MS-MS method was validated according to the FDA "Guidance for Industry Bioanalytical Method Validation".

Plasma was diluted 1:10 in 20mM Tris + 1% formic acid (FA) and incubated for 5 hours at room temperature. After incubation samples were transferred to LC-MS vials and kept at 4°C in the tray. Samples were analyzed on a Liquid Chromatography system LC20 AVP (Shimadzu, Japan) connected to an API3000™ mass spectrometer (AbSciex, USA). 5µl of diluted sample was injected onto a KromasilCAAP48 an immunomodulatory sepsis biomarker Eternity-2.5-C18 2.1x100mm UHPLC column (Dichrom GmbH, Marl, Germany) with a precoupled Krud Katcher Ultra HPLC In Line Filter 0.5µm (Phenomenex, USA). The peptide was eluted with solvent A (H2O + 0.1% FA) and solvent B (AcN + 0.1% FA), starting at 90% A for 2 min, followed by a 6 min linear gradient to 40% A, 5 min 40% A, 1 min linear gradient to 90% A, 6 min 90% A with a flow rate of 200 µl/min. The column oven was set 38°C. Two peptides were chosen for fragmentation: the 5fold charged CAAP48 peptide (4789.7 Da) at 958.9 Da and the 6 fold charged CAAP48 peptide at 799.3 Da.
The following transitions could be detected at high intensities: 799.3/873.6, 799.3/243.4, 799.3/314.3, 958.9/1091.8. MS parameters were CXP:27, DP:60, EP:10, CAD:12, CUR:22, GS1:40:, GS2:50, IS:5500, TEM:600. CE was optimized for the single fragments (874.4: 27, 1091.5: 33, 243.4: 39, 314.3: 28). For detection of the CAAP47 peptide (4775.7 Da) equivalent transitions were chosen at 797.0/873.6, 797.0/243.4, 956.167/1088.4 and 797.0/314.3.

Calibrants were created by serially diluting synthesized CAAP48 peptide and CAAP47 peptide in 40g/l albumin solution (20% Albunorm Humanalbumin, Octapharm, Langenfeld, Germany, diluted in H2Obd). Samples were measured in duplicates.

### Example 3: Method validation

Method validation was carried out on the basis of the FDA "Guidance for Industry Bioanalytical Method Validation". Shortly, linearity was determined measuring 6 consecutively diluted standards 10 times and generating a linear standard curve with the mean values. Limit of detection (LOD) was determined to be the mean of 10 blank values plus 2 standard deviations (SD). The lower limit of quantification (LLOQ) was determined to be the lowest concentration that could be measured with acceptable precision (CV < 20%). Intra- and interday precision and accuracy were determined measuring three samples at 6.4, 11.4 and 36 ng/µl 8 times a day and 10 consecutive days, respectively. Mass spectrometric selectivity is defined as a constant ratio of quantifier transition (243.4) to qualifier transition (1091). Recovery was determined by spiking three standard concentrations in EDTA plasma of donor plasma. Autosampler stability was determined by measuring three samples 8 times in a time range of 24 hours. Freeze/thawCAAP48 an immunomodulatory sepsis biomarker stability was determined by thawing and freezing two samples on 8 consecutive days. Parallelism was determined by diluting a sample consecutively in buffer and referring the concentrations of the dilutions to the original concentration.

Validation resulted in a linear calibration curve in a range of 2-200ng/µl with a coefficient of determination of 1. The LOD was 2.03 ng/µl, the LLOQ was about 6 ng/µl (interday precision = 15% at 6.4 ng/µl). Intraday accuracy was determined to be 0.98, 1.13 and 0.88 for the three respective concentrations. Interday accuracy was determined to be 1.07, 1.01 and 0.95. Intraday precision was 4, 11 and 11%, interday precision 15, 13 and 10%. Specificity, defined as the transition ratio of 243/1091, had a CV of 15%. Autosampler stability for 24 hours was given with CVs of 13, 11 and 11%. Two samples at 7 and 47 ng/µl were thawed and refrozen on eight consecutive days, without relevant impact on peptide concentration (CV 9 and 5%). Mean recovery of 1, 5 and 10 ng CAAP48 peptide was 87.4, 70.5 and 89.6%, respectively. Parallelism was defined as the deviation for two 1:2 dilutions of the 36 ng/µl sample, which was 4.91 and 7.32%.

### Example 4: Statistics and data analysis

Peak areas, calibration curves and concentrations were calculated using Analyst 1.51 (AbSciex, USA). Statistical analysis including testing for significance and ROC-curve analysis was carried out with SPSS 19 (IBM, USA). Normal distribution of data was checked for each patient group. If data showed normal distribution the parametric student t-test for two independent samples was used, otherwise the non-parametric Mann-Whitey-u-test for two independent samples was applied for determination of statistical significance. Cut-Off values from ROC-curves were determined using the Youden index.

Determination of significant differences between the areas under two independent ROC curves was performed according to Hanley and McNeil. For the cell experiments three to five independent replicates using cells from non-identical donors were analyzed. The level of significance was 0.05.

### Figure legend

**Fig. 1****: CAAP47/48 concentrations are significantly elevated in the blood of patients suffering from a bacterial infection.**
   CAAP47/48 concentrations are significantly higher in patients suffering from a bacterial infection compared to patients suffering from a viral infection. The median concentration for bacterial infection is 7.97 µM, which is a 4-fold increase compared to patients with a viral infection.
**Fig. 2****: Cleavage sites in the AAT reactive center loop (RCL).**
   Shown are the known cleavage sites in the RCL (bold) of AAT with respective enzymes gathered from the MEROPS database. Human proteases are displayed in black, proteases of other species in grey. Also for every cleavage site the respective molecular weight of the C-terminal peptide is indicated. Cleavage between Phe352-Leu353 leads to the generation of CAAP48 (red frame). However, in the presence of SNP rs1303 cleavage at this site leads to the generation of CAAP47, a peptide with an E > D substitution at the position indicated in blue.
**Fig**. **3**: **Median CAAP47/48 concentrations are significantly lower in the blood of patients suffering from a fungal infection than in patients suffering from a bacterial infection.**
   CAAP47/48 concentrations are significantly higher in patients suffering from a bacterial infection compared to patients suffering from a fungal infection. The median concentration for fungal infection is 4.5 µM.

## Claims

1. A method for determining whether in a patient an infection is of bacterial or non-bacterial origin, the method comprising:
(a) determining the concentration of at least one antitrypsin (ATT) fragment comprising CAAP48 and/or CAAP47, in a sample that has been obtained from said patient, and
(b) determining whether said patient suffers from an infection of bacterial or non-bacterial origin based on the concentration of at least one ATT fragment comprising CAAP48 and/or CAAP47 and designating the infection as of bacterial or non-bacterial origin,
wherein said non-bacterial infection is selected from the group comprising viral and fungal infections.

2. A method according to claim 1, wherein if the concentration of said at least one ATT fragment comprising CAAP48 and/or CAAP47 is above a certain cut-off value, the infection is designated as of bacterial origin.

3. A method according to claim 1, wherein if the concentration of said at least one ATT fragment comprising CAAP48 and/or CAAP47 is below a certain cut-off value, the infection is designated as of non-bacterial origin, wherein said non-bacterial origin is selected from the group comprising viral and fungal origins.

4. A method according to claim 1 or 2, wherein, if the concentration of said at least one ATT fragment comprising CAAP48 and/or CAAP47 is at least 2.5-fold higher than a reference concentration, the infection is designated as of bacterial origin.

5. A method according to any of the preceding claims, wherein said sample is selected from a group comprising a plasma sample, a serum sample, a whole blood sample, a blood sample or fractions thereof, a lymphatic fluid sample, a urine sample and an extract of any of the aforementioned samples.

6. A method according to any of the preceding claims, wherein said at least one ATT fragment comprising CAAP48 and/or CAAP47, is CAAP48 and/or CAAP47.

7. A method according to claim 6, wherein the cut-off value for determining that an infection is of bacterial origin is a concentration of CAAP48 and/or CAAP47 of at least 5 µM in the blood of said patient and may deviate depending on the patient analyzed by about 20 %.

8. A method according to claim 6, wherein, if the concentration of said CAAP48 and/or CAAP47 is at least 2.5 fold higher than a reference concentration, the infection is designated as of bacterial origin.

9. A kit for determining whether an infection is of bacterial or non-bacterial origin, comprising:
(a) means for determining the concentration of at least one ATT fragment comprising CAAP48 and CAAP47 in a sample,
(b) means for comparing the determined concentration of said at least one ATT fragment comprising CAAP48 and CAAP47 with reference concentrations, and
(c) instructions for carrying out the method and correlating the determined concentrations with the type of infection.

10. A kit according to claim 9, wherein the means for determining the concentration of said at least one ATT fragment comprising CAAP48 and CAAP47, is an antibody specific for at least one ATT fragment comprising CAAP48 and CAAP47.

11. A kit according to claim 10, wherein said fragment is CAAP48 and CAAP47 and said antibody is specific for CAAP48 and CAAP47.

12. A kit according to any of the claims 9 to 11, wherein the means for comparing the determined concentrations comprise standard data showing the correlation between the concentration of said at least one ATT fragment comprising CAAP48 and CAAP47 contained in samples and the origin of infection.

13. A kit according to claims 9 to 12, wherein an infection of non-bacterial origin is an infection selected from the group comprising viral and fungal infections.

## Patentansprüche

1. Verfahren zur Bestimmung, ob bei einem Patienten eine Infektion bakteriellen oder nichtbakteriellen Ursprungs ist, wobei das Verfahren umfasst:
(a) Bestimmen der Konzentration von mindestens einem Antitrypsin (ATT)-Fragment, umfassend CAAP48 und/oder CAAP47, in einer Probe, die von dem Patienten erhalten wurde, und
(b) Bestimmen, ob der Patient an einer Infektion bakteriellen oder nicht bakteriellen Ursprungs leidet, basierend auf der Konzentration mindestens eines ATT-Fragments, das CAAP48 und/oder CAAP47 umfasst, und Bezeichnen der Infektion als bakteriellen oder nichtbakteriellen Ursprungs;
wobei die nichtbakterielle Infektion ausgewählt ist aus der Gruppe umfassend Virus- und Pilzinfektionen.

2. Verfahren nach Anspruch 1, wobei, wenn die Konzentration des mindestens einen ATT-Fragments, umfassend CAAP48 und/oder CAAP47, über einem bestimmten Grenzwert liegt, die Infektion als bakteriellen Ursprungs bezeichnet wird.

3. Verfahren nach Anspruch 1, wobei, wenn die Konzentration des mindestens einen ATT-Fragments, umfassend CAAP48 und/oder CAAP47, unter einem bestimmten Grenzwert liegt, die Infektion als nichtbakteriellen Ursprungs bezeichnet wird, wobei der nichtbakterielle Ursprung ausgewählt ist aus der Gruppe umfassend viralen und pilzlichen Ursprung.

4. Verfahren nach Anspruch 1 oder 2, wobei, wenn die Konzentration des mindestens einen ATT-Fragments, umfassend CAAP48 und/oder CAAP47, mindestens 2,5-fach höher als eine Referenzkonzentration ist, die Infektion als bakteriellen Ursprungs bezeichnet wird.

5. Verfahren nach einem der vorhergehenden Ansprüche, wobei die Probe ausgewählt ist aus einer Gruppe umfassend eine Plasmaprobe, eine Serumprobe, eine Vollblutprobe, eine Blutprobe oder Fraktionen davon, eine Lymphflüssigkeitsprobe, eine Urinprobe und einen Extrakt einer der oben genannten Proben.

6. Verfahren nach einem der vorhergehenden Ansprüche, wobei das mindestens eine ATT-Fragment, das CAAP48 und/oder CAAP47 umfasst, CAAP48 und/oder CAAP47 ist.

7. Verfahren nach Anspruch 6, wobei der Grenzwert zum Bestimmen, dass eine Infektion bakteriellen Ursprungs ist, eine Konzentration von CAAP48 und/oder CAAP47 von mindestens 5 µM im Blut des Patienten ist und, abhängig von dem analysierten Patienten, um etwa 20% abweichen kann.

8. Verfahren nach Anspruch 6, wobei, wenn die Konzentration des CAAP48 und/oder CAAP47 mindestens 2,5-fach höher als eine Referenzkonzentration ist, die Infektion als bakteriellen Ursprungs bezeichnet wird.

9. Ein Kit zur Bestimmung, ob eine Infektion bakteriellen oder nichtbakteriellen Ursprungs ist, umfassend:
(a) Mittel zum Bestimmen der Konzentration von mindestens einem ATT-Fragment, umfassend CAAP48 und CAAP47, in einer Probe,
(b) Mittel zum Vergleichen der bestimmten Konzentration des mindestens einen ATT-Fragments, umfassend CAAP48 und CAAP47, mit Referenzkonzentrationen, und
(c) Anweisungen zur Durchführung der Methode und zur Korrelation der ermittelten Konzentrationen mit der Art der Infektion.

10. Kit nach Anspruch 9, wobei das Mittel zum Bestimmen der Konzentration des mindestens einen ATT-Fragments, das CAAP48 und CAAP47 umfasst, ein Antikörper ist, der für mindestens ein ATT-Fragment, das CAAP48 und CAAP47 umfasst, spezifisch ist.

11. Kit nach Anspruch 10, wobei das Fragment CAAP48 und CAAP47 ist und der Antikörper spezifisch für CAAP48 und CAAP47 ist.

12. Kit nach einem der Ansprüche 9 bis 11, wobei die Mittel zum Vergleichen der bestimmten Konzentrationen Standarddaten umfassen, die die Korrelation zwischen der Konzentration des mindestens einen ATT-Fragments, das CAAP48 und CAAP47 umfasst, das in Proben enthalten ist, und dem Infektionsursprung zeigen.

13. Kit nach den Ansprüchen 9 bis 12, wobei eine Infektion nichtbakteriellen Ursprungs eine Infektion ausgewählt aus der Gruppe umfassend Virus- und Pilzinfektionen ist.

## Revendications

1. Procédé pour déterminer si, chez un patient, une infection est d'origine bactérienne ou non bactérienne, le procédé comprenant :
(a) la détermination de la concentration d'au moins un fragment d'antitrypsine (ATT) comprenant la CAAP48 et/ou la CAAP47, dans un échantillon qui a été obtenu dudit patient, et
(b) la détermination si ledit patient souffre d'une infection d'origine bactérienne ou non bactérienne, sur la base de la concentration d'au moins un fragment d'ATT comprenant la CAAP48 et/ou la CAAP47, et la désignation de l'infection comme étant d'origine bactérienne ou non bactérienne,
dans lequel ladite infection non bactérienne est choisie dans le groupe comprenant les infections virales et fongiques.

2. Procédé selon la revendication 1, dans lequel, si la concentration dudit au moins un fragment d'ATT comprenant la CAAP48 et/ou la CAAP47 est supérieure à une certaine valeur de coupure, l'infection est désignée comme d'origine bactérienne.

3. Procédé selon la revendication 1, dans lequel, si la concentration dudit au moins un fragment d'ATT comprenant la CAAP48 et/ou la CAAP47 est inférieure à une certaine valeur de coupure, l'infection est désignée comme d'origine non bactérienne, ladite origine non bactérienne étant choisie dans le groupe comprenant les origines virales et fongiques.

4. Procédé selon la revendication 1 ou 2, dans lequel, si la concentration dudit au moins un fragment d'ATT comprenant la CAAP48 et/ou la CAAP47 est au moins 2,5 fois supérieure à une concentration de référence, l'infection est désignée comme d'origine bactérienne.

5. Procédé selon l'une quelconque des revendications précédentes, dans lequel ledit échantillon est choisi dans un groupe comprenant un échantillon de plasma, un échantillon de sérum, un échantillon de sang total, un échantillon de sang ou des fractions de ceux-ci, un échantillon de fluide lymphatique, un échantillon d'urine et un extrait d'un ou plusieurs quelconques des échantillons ci-dessus.

6. Procédé selon l'une quelconque des revendications précédentes, dans lequel ledit au moins un fragment d'ATT comprenant la CAAP48 et/ou la CAAP47 est la CAAP48 et/ou la CAAP47.

7. Procédé selon la revendication 6, dans lequel la valeur de coupure pour déterminer qu'une infection est d'origine bactérienne est une concentration de CAAP48 et/ou de CAAP47 d'au moins 5 µM dans le sang dudit patient, et peut s'en écarter, selon le patient analysé, d'environ 20 %.

8. Procédé selon la revendication 6, dans lequel, si la concentration de ladite CAAP48 et/ou de ladite CAAP47 est au moins 2,5 fois supérieure à une concentration de référence, l'infection est désignée comme d'origine bactérienne.

9. Trousse pour déterminer si une infection est d'origine bactérienne ou non bactérienne, comprenant :
(a) des moyens pour déterminer la concentration d'au moins un fragment d'ATT comprenant la CAAP48 et la CAAP47 dans un échantillon,
(b) des moyens pour comparer la concentration déterminée dudit au moins un fragment d'ATT comprenant la CAAP48 et la CAAP47 à des concentrations de référence, et
(c) des instructions pour mettre en œuvre le procédé et corréler les concentrations déterminées au type d'infection.

10. Trousse selon la revendication 9, dans laquelle le moyen pour déterminer la concentration dudit au moins un fragment d'ATT comprenant la CAAP48 et la CAAP47 est un anticorps spécifique d'au moins un fragment d'ATT comprenant la CAAP48 et la CAAAP47.

11. Trousse selon la revendication 10, dans laquelle le dit fragment est la CAAP48 et la CAAP47, et ledit anticorps est spécifique de la CAAP48 et de la CAAP47.

12. Trousse selon l'une quelconque des revendications 9 à 11, dans laquelle les moyens pour comparer les concentrations déterminées comprennent des données standard montrant la corrélation entre la concentration dudit au moins un fragment d'ATT comprenant la CAAP48 et la CAAP47 contenu dans les échantillons, et l'origine de l'infection.

13. Trousse selon les revendications 9 à 12, dans laquelle une infection d'origine non bactérienne est une infection choisie dans le groupe comprenant les infections virales et fongiques.
